Europäisches Patentamt

European Patent Office

Office européen des brevets

Publication number: **0 367 271**

**A2**

# EUROPEAN PATENT APPLICATION

Application number: **89120312.7**

Int. Cl.5 **A01N 1/02 , A61K 35/18**

Date of filing: **02.11.89**

Priority: **04.11.88 JP 280071/88**

Date of publication of application:
**09.05.90 Bulletin 90/19**

Designated Contracting States:
**DE FR GB IT**

Applicant: **SUMITOMO SEIKA CHEMICALS CO.,
LTD.**
**346-1, Miyanishi Harima-cho**
**Kako-gun Hyogo-ken(JP)**

Inventor: **Fukuda, Hirosuke Sumitomo Seika
Chemicals Co.,Ltd.**
**346-1, Miyanishi Harima-cho**
**Kako-gun Hyogo-ken(JP)**
Inventor: **Morimoto, Shigeru Sumitomo Seika
Chemicals Co.-Ltd**
**346-1, Miyanishi Harima-cho**
**Kako-gun Hyogo-ken(JP)**
Inventor: **Yamasaki, Eiko Sumitomo Seika
Chemicals Co.-Ltd.**
**346-1, Miyanishi Harima-cho**
**Kako-gun Hyogo-ken(JP)**
Inventor: **Saga, Kouichi Sumitomo Seika
Chemicals Co.-Ltd.**
**346-1, Miyanishi Harima-cho**
**Kako-gun Hyogo-ken(JP)**
Inventor: **Matsuzawa, Shigetaka**
**1-5-16, Motoizumi**
**Komae-shi Tokyo-to(JP)**
Inventor: **Seno, Taiko**
**3-5-16, Nakahama Joto-ku**
**Osaka-shi Osaka-fu(JP)**
Inventor: **Okubo, Yasuto**
**3-11-18, Taishi Ohji-cho**
**Kitakatsuragi-gun Nara-ken(JP)**

Representative: **Casalonga, Axel et al**
**BUREAU D.A. CASALONGA - JOSSE**
**Morassistrasse 8**
**D-8000 München 5(DE)**

## Improved red blood cell preservative solution.

An improved red blood cell preservative solution containing at least one component selected from the group consisting of glutathione, glutamine, cacodylic acid, cacodylates, barbituric acid, barbiturates, Good's buffer and Britton & Robinson's buffer in an amount of 1 to 200 mM.

## IMPROVED RED BLOOD CELL PRESERVATIVE SOLUTION

### FIELD OF THE INVENTION

The present invention relates to an improved red blood cell preservative solution, more particularly, a solution useful for preserving red blood cells for a long period of time.

### BACKGROUND OF THE INVENTION

In general, red blood cells used for transfusion or a test reagent for transfusion are preserved in various preservative solutions such as ACD solution composed of an anticoagulant and glucose, CPD solution which is ACD solution further containing a phosphate, Alsever's solution and the like. However, when red blood cells are preserved by using these known preservative solutions, a rise in hemolysis and browning of red blood cells are caused only within a month.

Then, there have been proposed various attempts and methods to improve these drawbacks. For example, Nihon Ishikai Zasshi (Journal of Japanese Medical Association), Vol. 75, No. 1, pages 15-28, 1976 discloses an attempt to add of adenine and inosine to prevent deterioration of red bloods cells during preservation. Japanese Patent Kokai No. 59-21621 discloses addition of sorbitol or xylitol. U.S. Patent No. 4,432,750 discloses addition of cholesterol. Japanese Patent Kokai No. 57-7419 discloses addition of albumin. According to these attempts and methods, a rise in hemolysis during preservation can be considerably inhibited. Further, as means for preventing browning of red blood cells, a treatment with carbon monoxide has been studied. However, even if these methods are employed, red blood cells are stably preserved, at most, for 1 to 3 months. When red blood cells are preserved for a more longer period of time, a rise in hemolysis and browning of red blood cells are yet caused. In Japanese Patent Kokai No. 1-199158, the present inventors disclose addition of certain water soluble polymers to Alsever's solution to improve preservation stability.

### OBJECTS OF THE INVENTION

The present inventors have studied to improve the above drawbacks of conventional red blood cell preservative solution. As a result, it has been found that deterioration caused during preservation such as lowering of agglutinability, a rise in hemolysis, browning of red blood cells and the like can be prevented by addition of a certain component to a conventional red blood cell preservative solution.

That is, the main object of the present invention is to provide an improved red blood cell preservative solution which can prevent the above deterioration of red blood cells during preservation for a long period of time, that is, a rise in hemolysis and browning of red blood cells and, further, maintain an antigen activity of red blood cell membrane for a long period of time.

This object as well as other objects and advantages of the present invention will become apparent to those skilled in the art from the following description.

### SUMMARY OF THE INVENTION

According to the present invention, there is provided an improved red blood cell preservative solution which comprises at least one component selected from the group consisting of glutathione, glutamine, cacodylic acid, cacodylates, barbituric acid, barbiturates, Good's buffers and Britton & Robinson's buffer in an amount of 1 to 200 mM.

### DETAILED DESCRIPTION OF THE INVENTION

Examples of cacodylates or barbiturates used in the present invention include their sodium and potassium salts and the like.

As Good's buffers. for example, the following compounds can be used:

N-Tris(hydroxymethyl)methyl-2-hydroxy-3-aminopropanesulfonic acid (hereinafter abbreviated as TAPSO),

N-Tris(hydroxymethyl)methyl-2-aminoethanesulfonic acid (hereinafter abbreviated as TES),

3-(N-Morpholino)propanesulfonic acid (hereinafter abbreviated as MOPS),

3-(N-Morpholino)-2-hydroxypropanesulfonic acid (hereinafter abbreviated as MOPSO),

Piperazin-N.N'-bis(2-ethanesulfonic acid) (hereinafter abbreviated as PIPES),

Piperazine-N.N'-bis(2-hydroxypropanesulfonic acid (hereinafter abbreviated as POPSO),

3-[N,N-Bis(2-hydroxyethyl)amino]-2-hydroxypropanesulfonic acid (hereinafter abbreviated as DIPSO),

N-2-Hydroxyethylpiperazine-N'-3-propanesulfonic acid (hereinafter abbreviated as EPPS),

N-2-Hydroxyethylpiperazine-N'-2-ethanesulfonic acid (hereinafter abbreviated as HEPES),

N-2-Hydroxyethylpiperazine-N'-2-hydroxypropane-3-sulfonic acid (hereinafter abbreviated as HEPPSO),

N-(2-Acetamido)-2-aminoethanesulfonic acid (hereinafter abbreviated as ACES),

N-(2-Acetamido) iminodiacetic acid (hereinafter abbreviated as ADA).

Usually. these compounds are used alone or in combination thereof in the form of an aqueous solution.

Britton & Robinson's buffer is an equimolar mixture of citric acid. potassium dihydrogen phosphate, boric acid and barbituric acid and. usually, these compounds are used as an aqueous solution containing them in a total concentration of not more than 160 mM (J. Chem. Soc., 1931, 458. 1456).

The amount of the above component to be used is. for example, 1 to 100 mM. preferably 10 to 80 mM in the case of glutathione: 1 to 200 mM. preferably 10 to 100 mM in the case of glutamine; 1 to 100 mM, preferably 5 to 80 mM in the case of cacodylic acid; 1 to 100 mM, preferably 10 to 80 mM in the case of barbituric acid; 1 to 150 mM. preferably 5 to 100 mM in the case of Good's buffers; 1 to 160 mM, preferably 20 to 160 mM in the case of Britton & Robinson's buffer.

The function and mechanism of the improvement of stability during preservation of red blood cells due to the above component is not clear at present. However, when the concentration of the component is lower than 1 mM. the effect of the addition of the component does not exert. On the other hand, when the concentration of the component is too high, osmotic pressure of the preservative solution increases, which give various hindrances to red blood cells.

The preservative solution of the present invention can be readily prepared by dissolving predetermined amounts of components of a conventional preserving solution and an predetermined amount of at least one component as described above in a distilled water and further adding a predetermined amount of NaCl thereto to adjust the resulting solution to a desired concentration and osmotic pressure. The components of a conventional preservative solution include those of Alsever's solution, ACD solution, CPD solution, CPDA solution, SAG solution, CPD-S solution, ADSOL solution, FRES solution and the like [i.e., buffers (e.g., phosphates, citrates, carbonates, etc.), antibiotics (e.g., chloramphenicol, neomycin, gentamycin, etc.), stabilizers (e.g., glucose, sucrose, maltose, lactose, mannitol, sorbitol, glucose-6-phosphate, ascorbates, tocopherol, dihydroxyacetone, cholesterol, bovine serum albumin, etc.) and the like].

The improved red blood cell preservative solution of the present invention has an excellent preservation stability. Thereby, a possible preservation period of 1 to 2 months provided by a conventional preservative solution can be extended up to 6 to 8 months. Therefore, the solution of the present invention is useful as a blood cell reagent as well as a preservative solution of blood cells for transfusion.

The following Examples and Comparative Examples further illustrate the present invention in detail but are not to be construed to limit the scope thereof.

Water used in the experiments was distilled water. Determination of preservation stability was carried out according to the following test method.

## Test method

Red blood cells were added to each preservative solutions in the concentration of 3% (v/v) and the mixture was preserved at 4° C. Evaluation of agglutinability was represented by the maximum dilution at which agglutination was observed by adding an antiserum to red blood cells antigen according to slide method.

Further, evaluation of hemolysis was carried out as follows. Red blood cells to be tested were added to aqueous 0.4% (w/v) NaCl solution and water, respectively, and each mixture was incubated at 37° C for 30 minutes. Then, the absorbance of each supernatant was measured. Namely, an amount of hemoglobin was represented by the absorbance and the rate of hemolysis was calculated by the following equation.

3

$$\text{Rate of hemolysis} = \frac{\text{Amount of hemoglobin in supernatant of 0.4\% NaCl solution}}{\text{Amount of hemoglobin in supernatant of a water}} \times 100$$

| Example 1 | |
|---|---|
| Component | Amount (g/l) |
| Glucose | 18.66 |
| Adenine hydrochloride | 0.676 |
| Inosine | 2.68 |
| Sodium citrate | 8.0 |
| Chloramphenicol | 0.5 |
| Neomycin | 0.3 |
| Gentamycin | 0.2 |
| Distilled water | up to 1 liter |

To Alsever s solution containing the antibiotics of the above formulation was added 80 mM HEPES and the mixture was adjusted to pH 7.4 to obtain a preservative solution (310 mOsm/kg, hereinafter referred to as the solution A) of the present invention. On the other hand, to Alsever's solution containing the antibiotics was added NaCl in a concentration of 0.31% (w/v) and the mixture was adjusted to pH 7.4 to obtain a control solution wherein osmotic pressure and pH were the same as those of the solution A.

Red blood cells were preserved in both solutions and samples of red blood cells collected immediately after preservation, after 3 months and after 6 months, respectively. Agglutinability and a rate of hemolysis of each sample were tested according to the manner as described above. The results are shown in Table 1.

As seen from Table 1, red blood cells preserved in the solution A has superior agglutinability and anti-hemolysis property in comparison with the case where red blood cells are preserved in the control solution. Particularly, regarding anti-hemolysis property, it is clear that anti-osmotic pressure property of red blood cell membrane is increased during preservation.

Table 1

| Preservative solution | Stability | | | | | |
|---|---|---|---|---|---|---|
| | Agglutinability (maximum dilution) | | | Rate of hemolysis | | |
| | 0 month | 3 months | 6 months | 0 month | 3 months | 6 months |
| Solution A | 256 | 256 | 128 | 40 | 20 | 20 |
| Control solution | 256 | 128 | 64 | 90 | 90 | 100* |
| Comparative Example 1 | 256 | 128 | 128 | 60 | 80 | 100* |
| (Note) | | . | | | | |

*: complete hemolysis

Comparative Example 1

According to the same manner as described in Example 1, a red blood cell preservative solution (310 mOsm/kg, pH 7.4) was obtained except that bovine serum albumin (5 g/l) and NaCl were added in the concentration of 0.30% (w v) in place of HEPES. The same control solution as that used in Example 1 was used. The same red blood cells as that used in Example 1 were preserved in these solutions and agglutinability and rate of hemolysis were tested.

The results are also shown in Table 1.

As seen from Table 1, regarding change of agglutinability with time, red blood cells in the preservative solution of Comparative Example 1 is almost the same as that preserved in the solution A of Example 1. However, it is clear that anti-hemolysis property of Comparative Example 1 is considerably deteriorated and is almost the same as that of the control solution.

| Example 2 | |
|---|---|
| Component | Amount (g/l) |
| Glucose | 13.3 |
| Sodium citrate | 13.2 |
| Citric acid | 4.8 |
| Distilled water | up to 1 liter |

ACD solution of the above formulation was diluted with the equal amount of water and to this were added chloramphenicol (0.5 g/l), neomycin (0.3 g/l) and gentamycin (0.2 g/l) to obtain a diluted ACD solution. To the diluted ACD solution was added 30 mM HEPES and adjusted to pH 7.4 to obtain a preservative solution (240 mOsm/kg, hereinafter referred to as the solution B) of the present invention. On the other hand, to the diluted ACD solution was added NaCl in the concentration of 0.12% (w/v) and adjusted to pH 7.4 to obtain a control solution wherein osmotic pressure and pH are the same as that of the solution B.

Then, according to the same manner as described in Example 1, the test was carried out to obtain the results as shown in Table 2.

As seen from Table 2, it is clear that the solution B has superior preservation stability in comparison with the control liquid.

Table 2

| Preservative solution | Stability | | | | | |
|---|---|---|---|---|---|---|
| | Agglutinability (maximum dilution) | | | Rate of hemolysis | | |
| | 0 month | 3 months | 6 months | 0 month | 3 months | 6 months |
| Solution B | 256 | 128 | 64 | 40 | 40 | 60 |
| Control solution | 256 | 64 | 32 | 90 | 100* | 100* |
| (Note) | | | | | | |

*: complete hemolysis

Examples 3 to 22

According to the same manner as described in Example 1, preservative solutions of the present invention and control solutions were obtained except that the components as shown in Table 3 were used in place of HEPES. According to the test method as described above, agglutinability and hemolysis were tested.

5

Table 3

| Ex. No. | Component (mM) | Total (mM) |
|---------|----------------|------------|
| 3 | glutathione (50) | 50 |
| 4 | glutamine (50) | 50 |
| 5 | cacodylic acid (40) | 40 |
| 6 | barbituric acid (40) . | 40 |
| 7 | Britton & Robinson's buffer (80) | 80 |
| 8 | TAPSO (20) | 20 |
| 9 | TES (40) | 40 |
| 10 | MOPS (60) | 60 |
| 11 | MOPSO (80) | 80 |
| 12 | PIPES (40) | 40 |

## Table 3 (continued)

| Ex. No. | Component (mM) | Total (mM) |
|---------|----------------|------------|
| 13 | POPSO (40) | 40 |
| 14 | DIPSO (40) | 40 |
| 15 | EPPS (50) | 50 |
| 16 | HEPPSO (50) | 50 |
| 17 | ACES (50) | 50 |
| 18 | ADA (40) | 40 |
| 19 | HEPES (80) + glutathione (50) | 130 |
| 20 | HEPES (80) + glutamine (70) | 150 |
| 21 | HEPES (80) + glutathione (50) + glutamine (50) | 180 |
| 22 | Britton & Robinson's buffer (80) + glutamine (50) | 130 |

Almost the same results as those shown in Tables 1 and 2 were observed. Thus, it is clear that the red blood cell preservative solution of the present invention has excellent preservation stability of red blood cells in comparison with a conventional preserving solution.

**Claims**

7

1. An improved red blood cell preservative solution which comprises at least one component selected from the group consisting of glutathione, glutamine, cacodylic acid, cacodylates, barbituric acid, barbiturates, Good's buffer and Britton & Robinson's buffer in an amount of 1 to 200 mM.

2. A red blood cell preservative solution according to claim 1, wherein glutathione is contained in an amount of 1 to 100 mM.

3. A red blood cell preservative solution according to claim 1, wherein glutamine is contained in an amount of 1 to 200 mM.

4. A red blood cell preservative solution according to claim 1, wherein cacodylic acid is contained in an amount of 1 to 100 mM.

5. A red blood cell preservative solution according to claim 1, wherein barbituric acid is contained in an amount of 1 to 100 mM.

6. A red blood cell preservative solution according to claim 1, wherein Good's buffer is contained in an amount of 1 to 150 mM.

7. A red blood cell preservative solution according to claim 1, wherein Britton & Robinson's buffer is contained in an amount of 1 to 160 mM.